# EUROPEAN PATENT APPLICATION

(11) **EP 1 752 145 A1**
(43) Date of publication of application: **14.02.2007**
(21) Application number: 05743246.0
(22) Date of filing: 27.05.2005
(51) Int. Cl.: A61K 31/192, A61K 31/22, A61K 31/366, A61K 31/40, A61K 31/404, A61K 31/47, A61K 31/505, A61K 45/00, A61P 3/06, A61P 3/10

(54) **THERAPEUTIC AGENT FOR HYPERLIPEMIA AND THERAPEUTIC AGENT FOR DIABETES**

(30) Priority: 01.06.2004 US 575405 P
(71) Applicant: Kowa Company. Ltd., Nagoya-shi, Aichi-ken 460-8625 (JP)
(72) Inventor: AOKI, Taro, 3590037 (JP); YAMAGUCHI, Junji, 1890022 (JP); SASAKI, Yusuke, 1890022 (JP)
(74) Representative: Hartz, Nikolai
(86) International application number: PCT/JP2005/009758
(87) International publication number: WO 2005/117853

(57) **Abstract**

A therapeutic agent for hyperlipidemia, diabetes, or hypertriglyceridemia containing an HMG-CoA reductase inhibitor and a carboxylic acid compound represented by the following formula (1) or a salt thereof as effective ingredients.

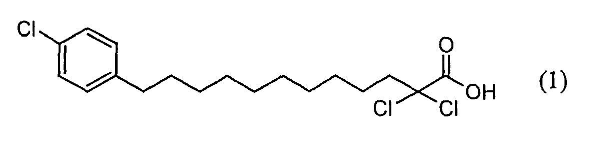

The therapeutic agent of the present invention has excellent effects of lowering blood cholesterol and blood triglycerides and is particularly useful for treatment of type IIb and type IV hyperlipidemia and treatment of diabetes and also useful for treatment of hypertriglyceridemia.

## Description

### Technical Field

The present invention relates to a therapeutic agent for hyperlipidemia and a therapeutic agent for diabetes having an excellent effect of lowering both blood cholesterol and blood triglycerides, and particularly to a therapeutic agent for hypertriglyceridemia.

### Background Art

Hyperlipidemia is a condition in which lipoprotein level in blood is abnormally elevated, and is strongly associated with diseases such as arteriosclerosis and myocardial infarction; therefore treatment of hyperlipidemia is thought to be important.
Various drugs are used for treatment of hyperlipidemia, and currently HMG-CoA reductase inhibitors such as lovastatin, simvastatin, pravastatin, fluvastatin, atorvastatin, rosuvastatin, and pitavastatin play a leading role in therapeutic agents therefor. Among them, pitavastatin ((3R,5S,6E)-7-[2-cyclopropyl-4-(4-fluorophenyl)-3-quinoly 1]-3, 5-dihydroxy-6-heptenoic acid) is known to have a strong inhibitory effect on HMG-CoA reductase and be useful as a hyperlipidemia agent (Patent Documents 1 to 3).

The maj or components of blood lipoprotein are cholesterol, triglycerides, and the like, and patients with hyperlipidemia not only show an increase in blood cholesterol, but also are often associated with an increase in blood triglycerides. When an HMG-CoAreductase inhibitor is administeredtopatients with hyperlipidemia, blood cholesterol is sufficiently reduced, whereas the blood triglyceride-lowering effect is not enough. A method of treatment in which an increased dose of an HMG-CoA reductase inhibitor is administered to hyperlipidemic patients with high levels of both blood cholesterol and blood triglycerides in order to sufficiently reduce the both levels causes a safety problem or the like and is therefore not being recommended.

On the other hand, diabetes is a metabolic abnormality, mainly involving sugar metabolism, due to an absolute or relative insufficiency of insulin secretion, and a prominent feature of diabetes is to cause hyperglycemia. Long-lasting hyperglycemia leads to complications in various organs that are mainly caused by angiopathy. Many cases of diabetes are associated with a rise in blood cholesterol and blood triglycerides.

On the other hand, a carboxylic acid compound represented by formula (1) below,

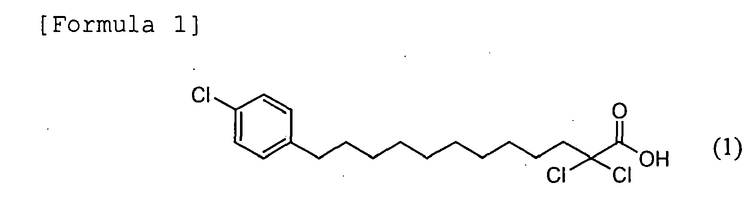

that is, 12-(4-chlorophenyl)-2,2-dichlorododecanoic acid or a salt thereof, is a therapeutic agent for diabetes and is known to improve conditions of hyperglycemia and hyperinsulinemia as well as to show effects of lowering blood cholesterol and blood triglycerides (Patent Documents 4 to 6).
However, there are many patients in whom elevation of both blood cholesterol and blood triglycerides cannot be fully suppressed only by a therapeutic agent for diabetes.
[Patent Document 1] Japanese Patent No. 2569746
[Patent Document 2] U.S. Patent No. 5102888
[Patent Document 3] European Patent No. 304063
[Patent Document 4] Japanese Patent No. 3012004
[Patent Document 5] U.S. Patent No. 968982
[Patent Document 6] European Patent No. 790824

### Disclosure of the Invention

The object of the present invention is to provide a therapeutic agent for hyperlipidemia and a therapeutic agent for diabetes having an excellent effect of lowering both blood cholesterol and blood triglycerides, and particularly a therapeutic agent for hypertriglyceridemia.

As a result of assiduous research in light of these circumstances, the present inventors came to perfect the present invention by discovering that combined use of an HMG-CoA reductase inhibitor and a carboxylic acid compound represented by formula (1) below (hereinafter, sometimes referred to as compound A) or a salt thereof

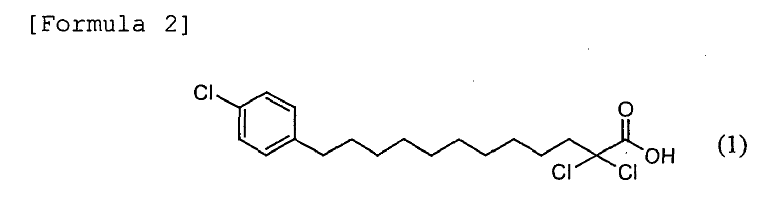

has an excellent effect of lowering both blood cholesterol and blood triglycerides and is useful for treatment of hyperlipidemia and diabetes, particularly for treatment of hypertriglyceridemia.

That is, the present invention provides a therapeutic agent for hyperlipidemia comprising an HMG-CoA reductase inhibitor and the compound A or a salt thereof as effective ingredients.
Further, the present invention provides a method for treatment of hyperlipidemia characterized in that effective doses of the HMG-CoA reductase inhibitor and the compound A or the salt thereof are administered.
Furthermore, the present invention provides use of the HMG-CoA reductase inhibitor and the compound A or the salt thereof for production of the therapeutic agent for hyperlipidemia.

Still further, the present invention provides a therapeutic agent for diabetes comprising the HMG-CoA reductase inhibitor and the compound A or the salt thereof as effective ingredients.
Still further, the present invention provides a method for treatment of diabetes characterized in that effective doses of the HMG-CoA reductase inhibitor and the compound A or the salt thereof are administered.
Still further, the present invention provides use of the HMG-CoA reductase inhibitor and the compound A or the salt thereof for production of the therapeutic agent for diabetes.

Still further, the present invention provides a therapeutic agent for hypertriglyceridemia comprising the HMG-CoA reductase inhibitor and the compound A or the salt thereof as effective ingredients.
Still further, the present invention provides a method for treatment of hypertriglyceridemia characterized in that effective doses of the HMG-CoA reductase inhibitor and the compound A or the salt thereof are administered.
Still further, the present invention provides use of the HMG-CoA reductase inhibitor and the compound A or the salt thereof for production of the therapeutic agent for hypertriglyceridemia.

The therapeutic agent of the present invention has excellent effects of lowering blood cholesterol and blood triglycerides and is particularly useful for treatment of type IIb and type IV hyperlipidemia and treatment of diabetes. Further, the therapeutic agent of the present invention is also useful for treatment of hypertriglyceridemia.

### Brief Description of the Drawings

Figure 1 is a graph showing lowering of blood triglycerides by combined administration of sodium salt of compound A and calcium salt of pitavastatin; and
Figure 2 is a graph showing lowering of blood triglycerides by combined administration of sodium salt of the compound A and calcium salt of atorvastatin.

### Best Mode for Carrying Out the Invention

HMG-CoA reductase inhibitors used in the present invention include all of the so-called statin compounds that have an inhibitory activity on cholesterol synthesis and are known as a therapeutic agent for hyperlipidemia. Lactone forms and ring-opened forms of these statins or salts thereof are all included. Hydrates and pharmaceutically-acceptable solvates of these compounds and salts thereof are included.
Preferred HMG-CoA reductase inhibitors include, for example, lovastatin (Japanese Patent Laid-Open No. 57-163374, U.S. Patent No. 4231938), simvastatin (Japanese Patent Laid-OpenNo. 56-122375, U.S. PatentNo. 4444784), pravastatin (Japanese Patent Laid-Open No. 57-2240, U.S. Patent No. 4346227), fluvastatin (National Publication of International Patent Application No. 60-500015, U.S. Patent No. 4739073), atorvastatin (Japanese Patent Laid-Open No. 3-58967, U.S. Patent Nos. 4681893 and 5273995), rosuvastatin (Japanese Patent Laid-Open No. 5-178841, U.S. Patent No. 5260440), pitavastatin (Japanese Patent No. 2569746, U.S. Patent No. 5102888, European Patent No. 304063), and salts thereof.
Salts of the HMG-CoA reductase inhibitors include alkaline metal salts, alkaline-earth metal salts, ammonium salts, alkylammonium salts, and the like.
As the HMG-CoA reductase inhibitor, atorvastatin, pitavastatin, and salts thereof are preferred, and pitavastatin and salts thereof are particularly preferred. As the salt, calcium salt and sodium salt are particularly preferred.

The compound A (12-(4-chlorophenyl)-2,2-dichlorododecanoic acid) and salts thereof used in the present invention are prepared according to a method disclosed, for example, in the specification of Japanese Patent No. 3012004.
The salts of the compound A include alkaline metal salts, alkaline-earth metal salts, ammonium salts, alkylammonium salts, and the like. Specifically, sodium salt, potassium salt, magnesium salt, calcium salt, ammonium salt, and tetramethylammonium salt are shown as examples. As the salt of the compound A, sodium salt and potassium salt are particularly preferred.

The drug of the present invention is used in a combination of the compound A or the salt thereof and the HMG-CoA reductase inhibitor and has an action of lowering blood triglycerides markedly compared when each of sodium salt of the compound A and the HMG-CoA reductase inhibitor is administered alone, for example, in evaluation using rat as shown in examples described below.

Accordingly, the therapeutic agent of the present invention is effective for treatment of a condition accompanied by a high blood triglyceride state, and particularly useful for treatment of hyperlipidemia and diabetes.

The dosage form of the therapeutic agent of the present invention can be appropriately selected depending on therapeutic purpose, and for example, any of powder, granule, dry syrup, tablet, capsule, and injection may be accepted. These dosage forms can be produced by mixing the above-described medicinal ingredients with pharmaceutically acceptable carriers according to a conventional formulation method known to persons skilled in the art.

When solid preparations are prepared, tablets, granules, powder, capsules, and the like can be produced according to a conventional method after adding excipient, and as necessary, binder, disintegrating agent, lubricant, coloring agent, taste-modifying agent, flavoring agent, and the like. These additives may be ones generally used in the field, and for example, lactose, sodium chloride, glucose, starch, microcrystalline cellulose, and silicic acid as the excipient, water, ethanol, propanol, simple syrup, gelatin solution, hydroxypropyl cellulose, methyl cellulose, ethyl cellulose, shellac, calcium phosphate, and polyvinylpyrrolidone as the binder, agar powder, sodium hydrogencarbonate, sodium lauryl sulfate, and stearic acid monoglyceride as the disintegrating agent, purified talc, stearic acid salt, borax, and polyethylene glycol as the lubricant, β-carotene, yellow iron sesquioxide, and caramel as the coloring agent, and saccharose and orange peel as the taste-modifying agent can be listed as examples.

When liquid oral preparations are prepared, oral solution, syrup, elixir, and the like can be produced according to a conventional method by adding taste-modifying agent, buffer agent, stabilizer, preservative, and the like. These additives may be ones generally used in the field, and for example, the taste-modifying agent includes saccharose; the buffer agent includes sodium citrate; the stabilizer includes tragacanth; and the preservative includes p-hydroxybenzoic ester.

When injections are prepared, hypodermic, intramuscular, and intravenous injections can be produced according to a conventional method by adding pH controlling agent, stabilizer, isotonizing agent, and the like. These additives may be ones generally used in the field, and for example, sodium phosphate as the pH controlling agent, sodium pyrosulfite as the stabilizer, and sodium chloride as the isotonizing agent can be listed as examples.

The dosage regimen for the therapeutic agent of the present invention is not particularly limited, and in addition to simultaneous administration of the both drugs, these may be separately administered at any interval. That is, theHMG-CoA reductase inhibitor and the compound A or the salt thereof may be used as a single preparation by mixing with pharmaceutically acceptable diluents, excipients, and the like or as a set of preparations in which the both drugs are separately formulated. When the both drugs are separately formulated, the both preparations may not be in the same dosage form.

Although the dose of the therapeutic agent of the present invention is appropriately selected depending on the condition, the HMG-CoA reductase inhibitor may be administered at 0.1 to 100 mg per day, preferably at 1 to 50 mg per day, and the compound A or the salt thereof may be administered at 0.1 to 500 mg per day, preferably at 1 to 100 mg per day based on the compound A. Further, the administration may be once a day or divided into two or more doses.

### Examples

Hereinafter, the present invention is more specifically explained based on examples. However, the present invention is not limited to these examples.

### Example 1

The effect of lowering blood triglycerides by combined administration of sodium salt of the compound A and calcium salt of pitavastatin was determined by the following method.

### 1. Test animal and rearing environment

Six-week-old male Wistar strain rats (Nippon Ikagaku Jikken Doubutsu Kabushiki Kaisha) were used for the test. Throughout the experimental period, the rats were fed in a rearing room maintained at a light-dark cycle (light period lit by room lamp: 7 am to 7 pm), a temperature of 23±3 degrees C, and a humidity of 55±15%, and were allowed free access to solid feed (CE-2: CLEA Japan, Inc.) and tap water.

### 2. Drug preparation

Calcium salt of pitavastatin and sodium salt of the compound A were suspended in an aqueous solution of 0.5% by mass of sodium carboxymethylcellulose (Iwai Chemicals Co., Ltd.) and prepared so that the dose amount became 2 mL/kg. Since calcium salt of pitavastatin contained 9.43% by mass of water, 1.1-fold by mass of the dose amount was weighed to correct. The suspension was refrigerated (4 degrees C) and kept in a light-resistant bottle, and its preparation was carried out every 7 days.

### 3. Test method

Thirty-two rats were divided into four groups (eight rats per each group) so that blood total cholesterol and blood triglycerides are averaged. That is, the four groups consisted of a control group, a group of calcium salt of pitavastatin alone (10 mg/kg), a group of sodium salt of the compound A alone (1 mg/kg), and a group of a combination of calcium salt of pitavastatin (10 mg/kg) and sodium salt of the compound A (1 mg/kg). The drugs were orally administered once a day (at 4 pm) for 14 days repeatedly, and the control group received orally the aqueous solution of 0.5% by mass of sodium carboxymethylcellulose at 2 mL/kg. All the groups were fasted for 18 hours after the final administration. Then, blood was collected and blood triglycerides were determined.

### 4. Methods of statistical analysis and data processing

Multigroup comparison between the control group and drug-administered group was performed with Dunnett'smultiple comparison test after Bartlett's test for analysis of variance, and a significance level lower than 5% was considered to indicate significant difference.

### 5. Test result

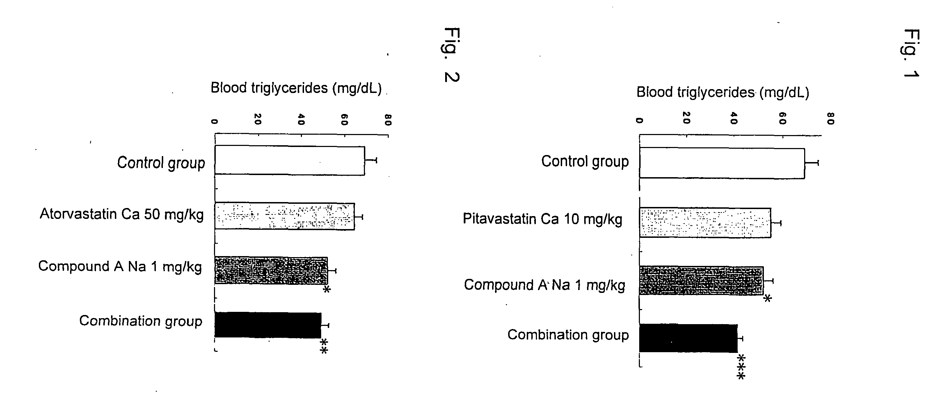

As shown in Figure 1 and Table 1, blood triglycerides of the groups administered with a single drug showed a tendency to be lowered in the group of calcium salt of pitavastatin alone and a significant reduction in the group of sodium salt of the compound A alone (p<0.05) in comparison with the control group. On the other hand, the effect of lowering blood triglycerides was greatly enhanced in the group of the combination of the both drugs (p<0.001), and their effects were synergistic.

**[Table 1]**

| Combination of pitavastatin and compound A | |
|---|---|
| | Reduction rate of blood triglycerides (%) |
| Control group | 0 |
| Pitavastatin Ca 10 mg/kg | 20 |
| Compound A Na 1 mg/kg | 25 |
| Combination group | 41 |

### Example 2

The effect of lowering blood triglycerides by combined administration of sodium salt of the compound A and calcium salt of atorvastatin was determined as in Example 1.
In place of calcium salt of pitavastatin in Example 1, calcium salt of atorvastatin was used, and the rats were divided into four groups: a control group, a group of calcium salt of atorvastatin (50 mg/kg), a group of sodium salt of the compound A (1 mg/kg), and a group of a combination of calcium salt of atorvastatin (50 mg/kg) and sodium salt of the compound A (1 mg/kg), and tested, the results of which are shown in Figure 2 and Table 2.

Blood triglycerides of the groups administered with a single drug showed a tendency to be lowered in the group of calcium salt of atorvastatin alone and a significant reduction in the group of sodium salt of the compound A alone (p<0.05) in comparison with the control group. On the other hand, the effect of lowering blood triglycerides was enhanced in the group of the combination of the both drugs (p<0.01).

**[Table 2]**

| Combination of atorvastatin and compound A | |
|---|---|
| | Reduction rate of blood triglycerides (%) |
| Control group | 0 |
| Atorvastatin Ca 50 mg/kg | 7 |
| Compound A Na 1 mg/kg | 25 |
| Combination group | 29 |

## Claims

1. A therapeutic agent for hyperlipidemia comprising as effective ingredients:
an HMG-CoA reductase inhibitor; and
a carboxylic acid compound represented by the following formula (1) or a salt thereof.

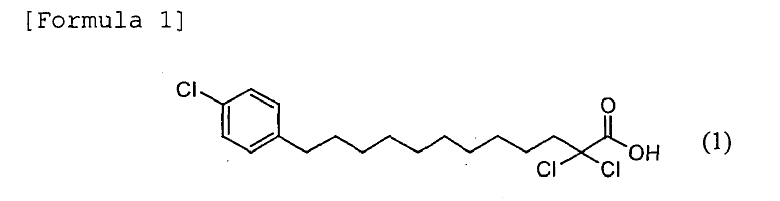

2. The therapeutic agent for hyperlipidemia according to claim 1, wherein the HMG-CoA reductase inhibitor is selected from lovastatin, simvastatin, pravastatin, fluvastatin, atorvastatin, rosuvastatin, pitavastatin, and salts thereof.

3. The therapeutic agent for hyperlipidemia according to claim 2, wherein the HMG-CoA reductase inhibitor is selected from atorvastatin, pitavastatin, and salts thereof.

4. The therapeutic agent for hyperlipidemia according to claim 3, wherein the HMG-CoA reductase inhibitor is pitavastatin or a salt thereof.

5. A method for treatment of hyperlipidemia **characterized in that** effective amounts of an HMG-CoA reductase inhibitor and a carboxylic acid compound represented by the following formula (1) or a salt thereof are administered.

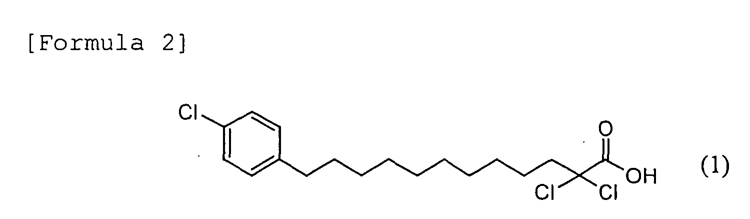

6. The method for treatment of hyperlipidemia according to claim 5, wherein the HMG-CoA reductase inhibitor is selected from lovastatin, simvastatin, pravastatin, fluvastatin, atorvastatin, rosuvastatin, pitavastatin, and salts thereof.

7. The method for treatment of hyperlipidemia according to claim 6, wherein the HMG-CoA reductase inhibitor is selected from atorvastatin, pitavastatin, and salts thereof.

8. The method for treatment of hyperlipidemia according to claim 7, wherein the HMG-CoA reductase inhibitor is pitavastatin or a salt thereof.

9. Use of an HMG-CoA reductase inhibitor and a carboxylic acid compound represented by the following formula (1) or a salt thereof for production of a therapeutic agent for hyperlipidemia.

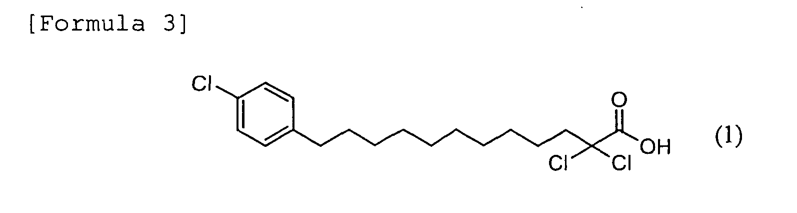

10. The use according to claim 9, wherein the HMG-CoA reductase inhibitor is selected from lovastatin, simvastatin, pravastatin, fluvastatin, atorvastatin, rosuvastatin, pitavastatin, and salts thereof.

11. The use according to claim 10, wherein the HMG-CoA reductase inhibitor is selected from atorvastatin, pitavastatin, and salts thereof.

12. The use according to claim 11, wherein the HMG-CoA reductase inhibitor is pitavastatin or a salt thereof.

13. A therapeutic agent for diabetes comprising as effective ingredients:
an HMG-CoA reductase inhibitor; and
a carboxylic acid compound represented by the following formula (1) or a salt thereof.

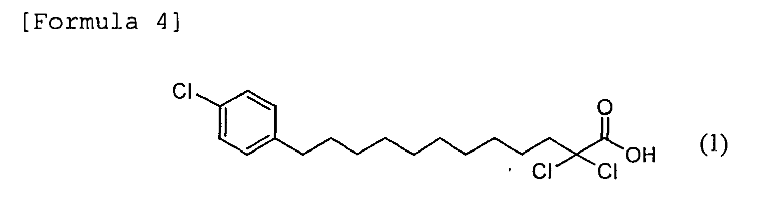

14. The therapeutic agent for diabetes according to claim 13, wherein the HMG-CoA reductase inhibitor is selected from lovastatin, simvastatin, pravastatin, fluvastatin, atorvastatin, rosuvastatin, pitavastatin, and salts thereof.

15. The therapeutic agent for diabetes according to claim 14, wherein the HMG-CoA reductase inhibitor is selected from atorvastatin, pitavastatin, and salts thereof.

16. The therapeutic agent for diabetes according to claim 15, wherein the HMG-CoA reductase inhibitor is pitavastatin or a salt thereof.

17. A method for treatment of diabetes **characterized in that** effective amounts of an HMG-CoA reductase inhibitor and a carboxylic acid compound represented by the following formula (1) or a salt thereof are administered.

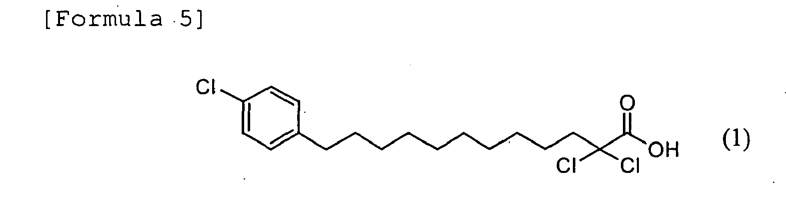

18. The method for treatment of diabetes according to claim 17, wherein the HMG-CoA reductase inhibitor is selected from lovastatin, simvastatin, pravastatin, fluvastatin, atorvastatin, rosuvastatin, pitavastatin, and salts thereof.

19. The method for treatment of diabetes according to claim 18, wherein the HMG-CoA reductase inhibitor is selected from atorvastatin, pitavastatin, and salts thereof.

20. The method for treatment of diabetes according to claim 19, wherein the HMG-CoA reductase inhibitor is pitavastatin or a salt thereof.

21. Use of an HMG-CoA reductase inhibitor and a carboxylic acid compound represented by the following formula (1) or a salt thereof for production of a therapeutic agent for diabetes.

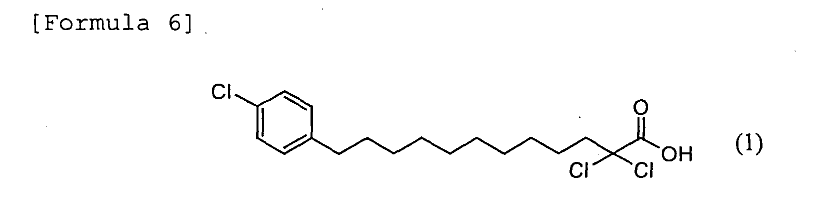

22. The use according to claim 21, wherein the HMG-CoA reductase inhibitor is selected from lovastatin, simvastatin, pravastatin, fluvastatin, atorvastatin, rosuvastatin, pitavastatin, and salts thereof.

23. The use according to claim 22, wherein the HMG-CoA reductase inhibitor is selected from atorvastatin, pitavastatin, and salts thereof.

24. The use according to claim 23, wherein the HMG-CoA reductase inhibitor is pitavastatin or a salt thereof.

25. A therapeutic agent for hypertriglyceridemia comprising as effective ingredients:
an HMG-CoA reductase inhibitor; and
a carboxylic acid compound represented by the following formula (1) or a salt thereof.

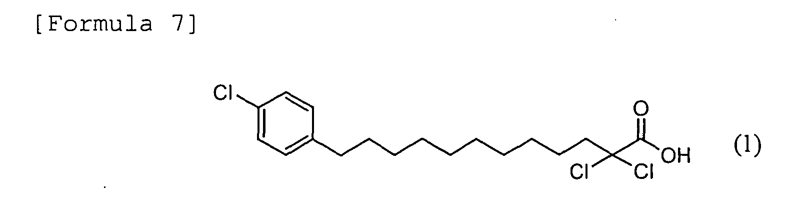

26. The therapeutic agent for hypertriglyceridemia according to claim 25, wherein the HMG-CoA reductase inhibitor is selected from lovastatin, simvastatin, pravastatin, fluvastatin, atorvastatin, rosuvastatin, pitavastatin, and salts thereof.

27. The therapeutic agent for hypertriglyceridemia according to claim 26, wherein the HMG-CoA reductase inhibitor is selected from atorvastatin, pitavastatin, and salts thereof.

28. The therapeutic agent for hypertriglyceridemia according to claim 27, wherein the HMG-CoA reductase inhibitor is pitavastatin.

29. A method for treatment of hypertriglyceridemia **characterized in that** effective amounts of an HMG-CoA reductase inhibitor and a carboxylic acid compound represented by the following formula (1) or a salt thereof are administered.

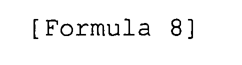

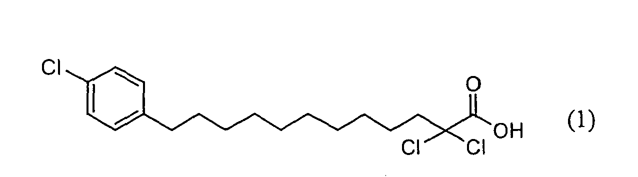

30. The method for treatment of hypertriglyceridemia according to claim 29, wherein the HMG-CoA reductase inhibitor is selected from lovastatin, simvastatin, pravastatin, fluvastatin, atorvastatin, rosuvastatin, pitavastatin, and salts thereof.

31. The method for treatment of hypertriglyceridemia according to claim 30, wherein the HMG-CoA reductase inhibitor is selected from atorvastatin, pitavastatin, and salts thereof.

32. The method for treatment of hypertriglyceridemia according to claim 31, wherein the HMG-CoA reductase inhibitor is pitavastatin or a salt thereof.

33. Use of an HMG-CoA reductase inhibitor and a carboxylic acid compound represented by the following formula (1) or a salt thereof for production of a therapeutic agent for hypertriglyceridemia.

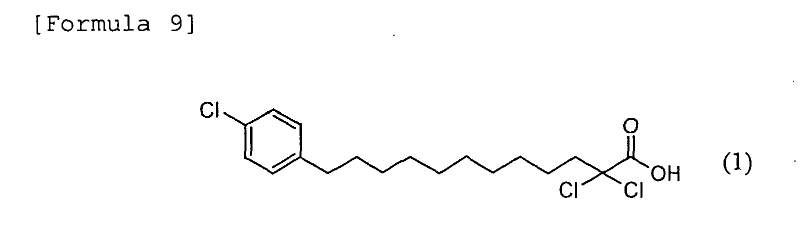

34. The use according to claim 33, wherein the HMG-CoA reductase inhibitor is selected from lovastatin, simvastatin, pravastatin, fluvastatin, atorvastatin, rosuvastatin, pitavastatin, and salts thereof.

35. The use according to claim 34, wherein the HMG-CoA reductase inhibitor is selected from atorvastatin, pitavastatin, and salts thereof.

36. The use according to claim 35, wherein the HMG-CoA reductase inhibitor is pitavastatin.
